(19) Europäisches Patentamt
European Patent Office
Office européen des brevets

(11) **EP 4 223 218 A1**

(12) **EUROPEAN PATENT APPLICATION**

(43) Date of publication:
**09.08.2023 Bulletin 2023/32**

(21) Application number: **22155099.9**

(22) Date of filing: **04.02.2022**

(51) International Patent Classification (IPC):
**A61B 5/026** (2006.01)   **A61B 5/00** (2006.01)

(52) Cooperative Patent Classification (CPC):
**A61B 5/026;** A61B 5/0066; A61B 2576/02

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **LUCASSEN, Gerhardus Wilhelmus**
  **Eindhoven (NL)**
- **DE BRUIJN, Frederik Jan**
  **Eindhoven (NL)**
- **SCHMEITZ, Harold Agnes Wilhelmus**
  **Eindhoven (NL)**

(74) Representative: **Philips Intellectual Property & Standards**
**High Tech Campus 52**
**5656 AG Eindhoven (NL)**

(54) **INTRAVASCULAR IMAGING DIAGNOSTICS**

(57)    There is provided a system and method for recommending a treatment of a vessel. The system is for use in conjunction with an intravascular imaging system which obtains images of the vessel and measures the position of each image along a longitudinal length of the vessel. The system comprises a processor adapted to receive a plurality of intravascular images of the vessel and respective positions along the vessel, determine, from the images, a cross sectional area of a section of the vessel at a plurality of positions along the vessel, determine, from the images and the respective position of the images along the vessel, a flow resistance of the vessel, and output a treatment recommendation based on the cross sectional area of the vessel and the flow resistance of the vessel.

EP 4 223 218 A1

FIG. 5

**Description**

FIELD OF THE INVENTION

The present invention relates to the field of intravascular imaging diagnostics, and in particular to the field of intravascular imaging diagnostics that provide a treatment recommendation based on a lesion in a vessel.

BACKGROUND OF THE INVENTION

Both coronary artery disease (CAD) and peripheral artery disease (PAD) are caused by atherosclerosis, which is the build-up of fatty plaque in the intima of the artery walls, narrowing, sometimes blocking, the arteries throughout the body, including the heart, brain, arms, legs, pelvis and kidneys. Plaque is made up of deposits of fat, cholesterol, and other substances. With the narrowing of arteries by plaque the blood flow is reduced.

CAD is currently diagnosed using electrocardiography, an echocardiogram, cardiac catherization, and of an angiogram and or computed tomography angiography (CTA), fractional flow reserve (FFR) or instantaneous wave free ratio or instant flow reserve (iFR). These assess whether stenosis is limiting blood flow in the arteries.

In peripheral venous disease (PVD), blood flow may be reduced not by plaque (as in the arteries) but by compression by nearby arteries (for example, the May-Thurner syndrome) or by thrombus formation. For example, deep vein thrombosis (DVT) produces a clot of blood cells, whereas over time, post-thrombotic syndrome (PTS) produces 'webbing' of a similar collagen-rich composition as the vessel wall.

PVD can be diagnosed using an ankle brachial index which measures the blood pressure in the lower legs relative to that in the arms. Alternatively, duplex ultrasonography can be used to measure to blood flow in an artery to see if there is a blockage. Other methods of visualising the blockages include CTA and magnetic resonance angiography.

Some methods of assessing CAD or PVD include intravascular (or intraluminal) imaging using optical coherence tomography (OCT) or intravascular ultrasound (IVUS) imaging. IVUS and OCT can be used pre-treatment for diagnosis of the 'target frames', exhibiting a minimum lumen diameter or area, and of 'reference frames' in an adjacent healthy vessel segment, for stent landing. These can also be used post-treatment, to check the stent placement and check for correct stent apposition.

The degree of CAD or PVD is assessed and a clinician then takes a decision about whether treatment is necessary. Treatment can include balloon angioplasty and/or applying a stent to treat the narrowing of the vessels.

With the estimated target vessel/lumen diameter or area and healthy reference diameter or areas and the lesion length the physician decides what stent size and length to use for treatment.

In IVUS and OCT currently stent selection is based on using vessel/lumen diameter and lesion length solely. Clinical evidence has shown that stent selection based on angiography alone gives worse outcome for the patient, so use of intravascular imaging is recommended.

Currently, in image-guided treatment of arterial and venous disease, the treatment plan is based on vessel or lumen diameter or area and lesion length estimates. This accounts for the majority of clinical cases where flow can be restored in the stenosed artery or vein.

Assessing whether treatment is necessary is inconsistent and difficult to do accurately. It is therefore desirable to provide an improved method assessing whether treatment is necessary.

SUMMARY OF THE INVENTION

The invention is defined by the claims.

According to the invention there is provided system for recommending the use or non-use of a stent in a vessel, the system for use in conjunction with an intravascular imaging system for vessels, the intravascular imaging system obtaining images of the vessel and measuring the position of each image along a longitudinal length of the vessel, the system comprising a processor, wherein the processor is adapted to: receive a plurality of intravascular images of the vessel at respective positions along the vessel; determine, from the images, a cross sectional area of a section of the vessel at a plurality of positions along the vessel; determine, from the images and the respective position of the images along the vessel, a flow resistance of the vessel; and output a treatment recommendation based on the cross sectional area of the vessel and the flow resistance of the vessel.

It is proposed to use both the cross sectional area of the vessel and the flow resistance as a basis for a treatment recommendation. This may provide a better (e.g. more accurate) overall assessment of the degree of stenosis. Furthermore, use of the proposed embodiments may ensure that decisions are consistent between patients and moreover, best practice is consistently applied.

Embodiments of the proposed invention may recommend treatment for a lesion which, although it does not heavily restrict the cross sectional area, extends a long way along the vessel. Lesion should be interpreted as a reduction in cross section of the vessel through which blood can flow, or with other words a stenosis or an abnormal narrowing of the vessel. The reduction of the cross section of the vessel can be caused by deposits on the vessel wall, such as fatty plagues, or by local compression of the vessel by adjacent portions of the anatomy. In contrast, embodiments may recommend that no treatment is applied to a lesion which heavily restricts the cross sectional area but is very short. Advantageously, this avoids medical procedures for both the patient and the clinician.

Proposed embodiments may be used together with current intravascular imaging systems and therefore not require a new imaging system. Embodiments of the proposed invention may be incorporated with existing/conventional intravascular imaging systems, thus providing new and/or extended functionality.

There is concern within the medical profession that stenting procedures are carried out unnecessarily and also that some patients who would benefit from the use of a stent are not receiving adequate treatment. Embodiments may address this problem by providing a treatment recommendation which is linked to the two key features of degree of stenosis and flow resistance.

The treatment recommendation may be based on at least one of the cross-sectional area being within a first predetermined range and the flow resistance being within a second predetermined range. The ranges provide guidance and a basis for the recommendation. However, there may be more than two ranges, and some of the ranges may be dependent on each other. There may be a decision tree comprising an assessment of whether the at least one of the cross-sectional areas is within a first predetermined range and the flow resistance is within a second predetermined range. If the amount of stenosis and flow resistance falls within a range, treatment may be recommended. Alternatively, treatment may be recommended if the amount of stenosis and flow resistance falls outside predetermined ranges.

The flow resistance may be calculated as a fraction of the flow resistance of the vessel, or a portion of the vessel, without lesions. Advantageously, this means that the amount of stenosis is based on the vessel, and the size of the vessel itself. Different vessels for different patients, or even for the same patient, may be different sizes so this ensures that the amount of stenosis is based on the actual vessel.

The flow resistance R may given by:

$$R = \frac{f}{x^2} R_0$$

where $R_0$ is the flow resistance of the vessel without a reduced cross section due to a lesion, f is the length of the section with reduced cross section, due to lesions, as a fraction of the length of the vessel and x is the reduced cross sectional area of the vessel as a fraction of the cross sectional area of the vessel being free from lesion. This provides a simple way of comparing the additional flow resistance due to the lesion, by taking into account both the amount of stenosis and the length of the stenosis.

The amount of stenosis may vary slightly along the length of the lesion and so the length of the section with reduced cross section may be defined as comprising the length of a section having a cross-sectional area within 10% of the reduced cross section.

The treatment recommendation may comprise recommending whether or not a stent should be used, but could also comprise outputting a recommendation for a particular stent. It could comprise outputting a recommendation for the size of the stent to be used.

According to the invention, there may be provided an intravascular imaging system comprising a system according to a proposed embodiment. Thus, embodiments may form part of the imaging system. The intravascular imaging system may be one or more of an intravascular ultrasound system and optical coherence tomography system.

The intravascular imaging system may comprise a motorized pullback device. Alternatively, or additionally it may comprise an X-ray imaging system for determining the position of a catheter tip. The motorized pullback device and/or the X-ray imaging system can be used to determine the position of the catheter tip when images are taken and therefore determine the position along the vessel represented by each image.

According to another aspect of the invention there is provided a computer implemented method for recommending the use or non-use of a stent, using images from an intravascular imaging system and positions of each image along a longitudinal length of the vessel, the method comprising receiving a plurality of intravascular images of the vessel, determining, from the images, a cross sectional area of the vessel at a plurality of positions along the vessel, determining, from the images and the respective position of the images along the vessel, a flow resistance of the vessel and outputting a treatment recommendation based on the cross sectional area of the vessel and the flow resistance of the vessel.

According to the invention there is provided a computer program comprising computer program code means which is adapted, when said computer program is run on a computer, to implement the method as described above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 depicts a first example of stenosis of a vessel;
Figure 2 depicts a second example of stenosis of a vessel;
Figure 3 depicts an imaging apparatus and system according to an embodiment of the invention;
Figure 4 depicts a process executed by the processor of Figure 3;
Figure 5 depicts a decision tree according to an embodiment the invention;
Figure 6 depicts a decision tree according to another embodiment of the invention; and
Figure 7 depicts a decision tree according to another embodiment of the invention; and
Figure 8 a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The present invention uses an overall assessment of the degree of severity of stenosis to output a recommendation. In particular, the present invention is not limited to the degree of stenosis at a particular position but measures the degree of stenosis at a plurality of positions.

Lesions, or stenosis, restricts the flow of blood and it is this which causes problems. Advantageously, the present invention takes account of this by calculating the flow resistance along the vessel and a recommendation based on this is presented.

Predetermined ranges of cross-sectional area and flow resistance can be set in order to determine whether a stent should be used. In this way, both the degree of stenosis and the length of stenosis along the vessel are taken into account. The predetermined ranges may be unique to the user, for example, the predetermined cross-sectional range may be set as a fractional value of the area of a healthy part of the vessel.

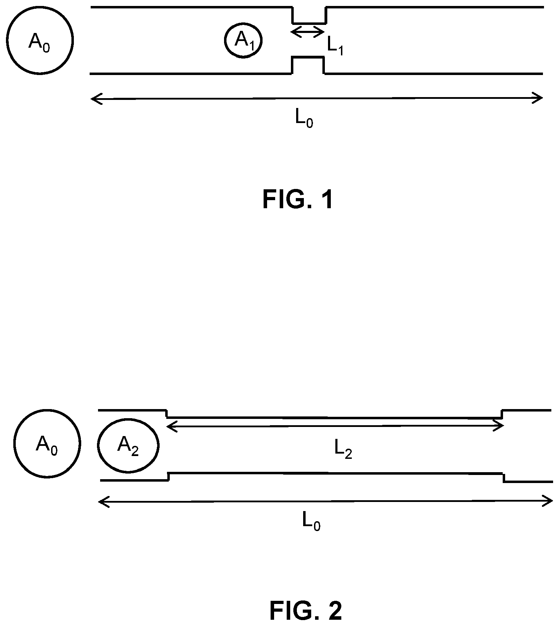

Figure 1 depicts a first example of stenosis of a vessel of cross sectional area Ao. In this example there is a high degree of stenosis but along only a short length of the vessel. The cross sectional area of the area of the vessel with the lesion is shown by $A_1$ and is 40% of Ao. The lesion is of length, not to scale, $L_1 = L_0/50$.

Figure 2 depicts a second example of stenosis of the same vessel. In contrast with the example of figure 1, there is just a small amount of stenosis but along a longer portion of the vessel. The cross sectional area of the area with the lesion is 70% of Ao and is shown by $A_2$ along a length of the lesion is $L_2$ where $L_2 = L_0/5$, not to scale.

In case that a practical rule of thumb would be used that when the estimated stenosis area is more than 50%, e.g. cross sectional area reduction, the physician would perform a stent placement, this would lead to suboptimal decision for treatment, as it will demonstrated below. There are situations where the restoring of blood flow in the larger vessels do not lead to improved flow downstream due to high vascular flow resistance. In the case of always stenting lesions with more than 50% area stenosis 'over-stenting' may be occurring in cases where the flow resistance is low enough to ensure proper flow. In case of never stenting under 50% area stenosis it might occur under-stenting when the flow resistance is so high that it impedes proper flow. There is therefore a desire to prevent over-stenting and under-stenting.

Assessing stenosis and treatment of the vessel depicted in figure 1 would lead to treatment by a stent due to the 60% stenosis, e.g. cross sectional area reduction, whereas the vessel in figure 2 may remain untreated because there is only 30% stenosis, e.g. cross sectional area reduction. However, due to the length of the lesion in the vessel of figure 2 the flow resistance may be higher.

Flow resistance along a vessel with no stenosis is given by:

$$R_0 = 8 \pi \eta L_0 / A_0^2$$

The flow resistance of the vessel depicted in figure 1, compared to Ro is given by:

$$R_1 = 8 \pi \eta L_1 / A_1^2 = 8 \pi \eta L_0 / (50 * 0.4 * 0.4 A_0^2) = R_0/8 = 0.125 * R_0$$

The flow resistance of the vessel depicted in figure 2, compared to Ro is given by:

$$R_2 = 8 \pi \eta L_2 / A_2^2 = 8 \pi \eta L_0 / (5*0.7*0.7 A_0^2) = R_0/(5*0.49) \approx 0.4 * R_0$$

As can be seen from these calculations, the lesion depicted in figure 2 results in a greater flow resistance than the lesion depicted in figure 1. A treatment decision based on flow resistance alone may stent the vessel depicted in figure 2, but leave the vessel of figure 1 untreated.

The present invention is used in conjunction with an imaging system 30, for example and IVUS or OCT imaging system, or a combination thereof. The imaging system obtains images of the vessel and measures the location of each image along a longitudinal position along the length of the vessel. The present invention comprises a system comprising a processor 10 which receives the plurality of intravascular images of the vessels.

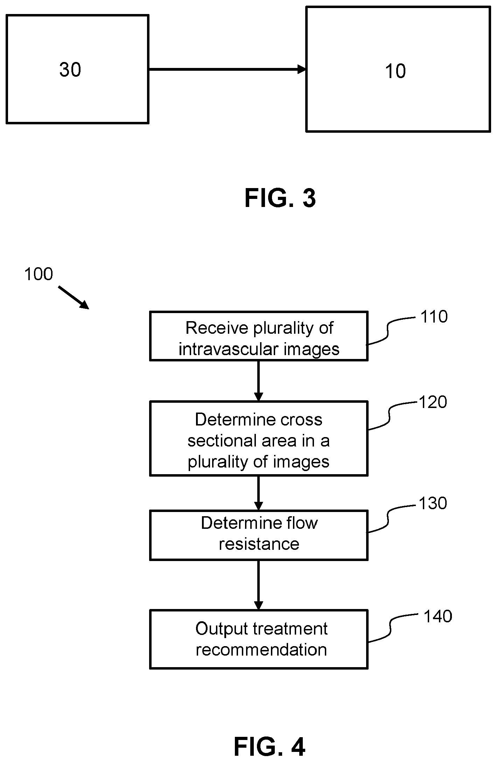

Figure 4 depicts the process carried out by the processor. Initially, the processor receives 110 the plurality of intravascular images of the vessel, together with the location along the vessel at which each of the images was taken.

The processor then determines 120 a cross sectional area of the vessel in a plurality of the images. The processor may determine the cross sectional area of the vessel in every image. Alternatively, the processor may initially determine the cross sectional area of every n-th image. When the cross section is consistent between images, the interim (or with other words, temporary) images may not be processed but where the cross section varies, the cross section for interim images may also be determined.

The processor may determine a cross sectional area of a portion of the vessel with no stenosis and use this as a reference area $A_R$. The cross sectional area of a portion of the vessel with a lesion, or stenosis may be determined as a fraction of this so that stenosis is expressed as a percentage. This means that the system is easily adapted to vessels of different sizes.

Using the determined cross sections and the position along the vessel the length of the lesion may be calculated. The lesion length may be the length of the vessel during which the cross sectional area varies by less than a predetermined amount. For example, length may be the length over which the cross sectional area varies by a predetermined percentage from the reference area $A_R$, which for example may be more than 10%. Alternatively, the length may be determined as the length of the portion of the vessel where the cross sectional is less than a predetermined percentage of the reference area $A_R$, used in the respective decision tree to arrive at a treatment decision.

Using the length of the lesion and the cross sectional area the flow resistance may be determined 130. This may be the absolute value of the flow resistance or it may be a relative, or a percentage value of the flow resistance with no lesions. To determine the relative flow resistance compared to the flow resistance of the vessel without a reduced cross section due to a lesion, the following equation may be used:

$$R = \frac{f}{x^2} R_0$$

where $R_0$ is the flow resistance of the vessel without a reduced cross section due to a lesion, f is the length of the section with reduced cross section, due to lesions, as a fraction of the length of the vessel and x is the reduced cross sectional area of the vessel as a fraction of the cross sectional area of the vessel being free from lesion.

In a further alternative, the processor may determine multiple portions of the vessel with a lesion or multiple adjacent lesions, wherein the stenosis or stenoses of the lesions may be determined as fractions of the reference area $A_R$ so that stenoses are expressed as percentages. The lesion lengths for each of the multiple portions of the lesion or multiple adjacent lesions are then determined for each of the multiple portions of the lesion or multiple adjacent lesions that has/have distinct cross sectional area with respect to the neighbouring portion of the lesion. The multiple lengths of the lesions reflect therefore the true lengths of the portions of the lesions that have the respective percentage of reduction of cross sectional areas with respective to the reference area $A_R$. The relative flow resistance due to a lesion or multiple adjacent lesions is determined compared to the flow resistance of the vessel without a reduced cross section by cumulative contributions of the multiple portions of the lesion or multiple adjacent lesions that has/have distinct cross sectional area with respect to the neighbouring portion of the lesion, ensuring that there is no underestimation or overestimation of the flow resistance due to the stenosis or stenoses caused by the lesion or multiple adjacent lesions. For the cumulative flow resistance computation the electrical resistance analogy can be used. In a further alternative, portions of the lesion or multiple adjacent lesions can be excluded from the computation of the cumulative contributions to the flow resistance in case the cross sectional area of the respective portion varies by less than a predetermined percentage from the reference area $A_R$, which predetermined percentage may be for example may be 10% or 20%.

Based on both the cross sectional area of the vessel and the flow resistance of the vessel a treatment recommendation is output 140. According to an example the treatment recommendation is whether or not the area should be stented. Alternatively, the treatment recommendation may be which stent, from a plurality of possible stents, to be used.

In more detail, the recommendation may be based on whether the cross-sectional area of the vessel is within a first predetermined range and/or whether the flow resistance is within a second predetermined range. Alternatively, it may be based on whether the cross section area of the vessel is within a first predetermined range and whether the flow resistance is within a second predetermined range, or whether the cross sectional area of the vessel is within a third predetermined range and the flow resistance is within a fourth predetermined range.

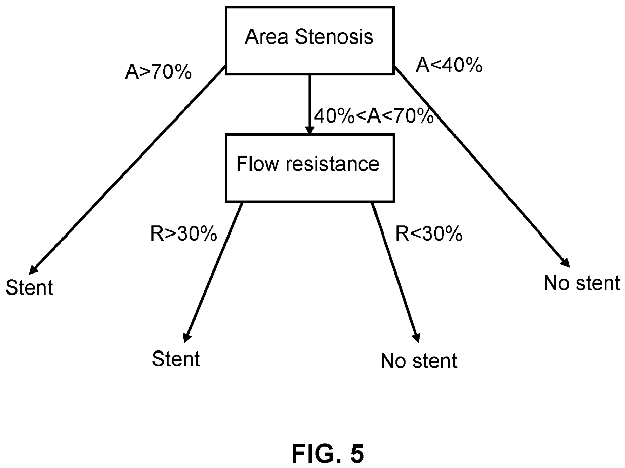

One example of the basis of the treatment recommendation is the use of a decision tree and such a decision tree is depicted in Figure 5. Inputs into the decision tree include the area stenosis and the flow resistance. As can be seen a decision is initially based on the area stenosis. If stenosis is above a predetermined value then a recommendation to treat the area using a stent is output, regardless of the flow resistance. If stenosis is less than 40% a recommendation not to stent the area is output, regardless of the flow resistance. If the area stenosis is between 40% and 70% the flow resistance is considered. If the flow resistance is more than 30% a recommendation to stent is output. If the flow resistance is less than 30% a recommendation not to stent is output. In this way the treatment recommendation is based not just on the area stenosis but also on the flow resistance. This provides improved decision making about whether or not to use a stent. It can therefore help avoid unnecessary medical procedures but also help ensure that medical intervention is taken where it would be beneficial.

The specific percentages used in Figure 5 are merely illustrative and not limiting. In one example, clinicians may be able to set the values. For example, different values may be used depending on the age of the patient.

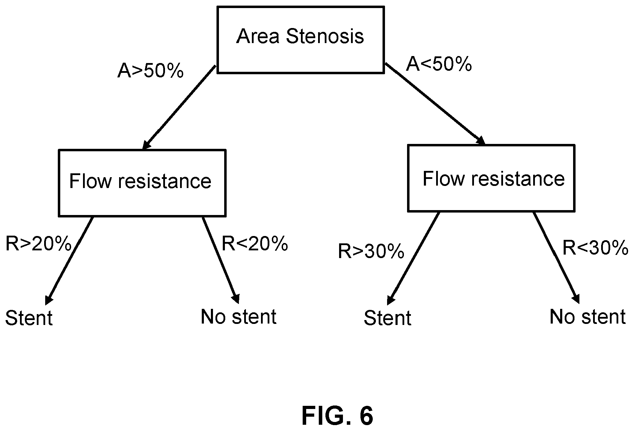

Figure 5 depicts one example decision tree according to an embodiment of the invention. In this example the initial step in the decision tree is based on area stenosis and for more extreme cases a recommendation can be output based on this alone. However, for cases other that the extreme cases the flow resistance is also used to output a recommendation. Figure 6 depicts an alternative decision tree. In this decision tree the amount of stenosis is used to determine which branch of the decision tree is used. As can be seen, the branch for the larger degree of stenosis has lower thresholds for flow resistance whereas the branch for the smaller degree of stenosis has higher thresholds for flow resistance. Specifically, the stenosis is assessed as being above or below 50% of the cross sectional area of the vessel. If the stenosis is greater than 50% a recommendation about whether or not to stent is based on whether the flow resistance is more than 20% of the vessel without lesions. If the flow resistance is more than 20% a recommendation to stent is output. If the flow resistance is less than 20% a recommendation not to stent is output. If the area stenosis is less than 50% the threshold flow resistance value is 30% so, if the flow resistance is greater than 30% a recommendation to stent will be output, but if the flow resistance is less than 30% a recommendation not to stent will be output.

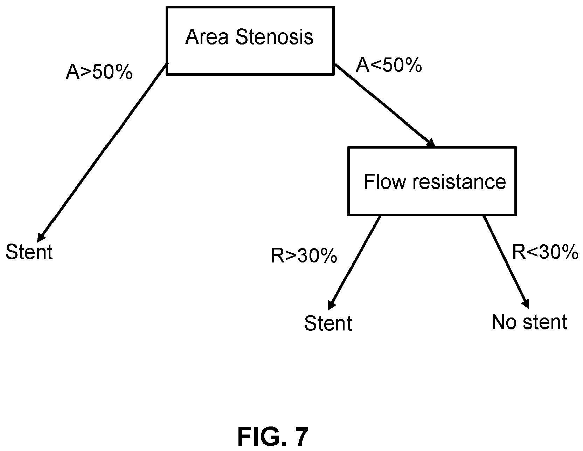

Figure 7 depicts another decision tree according to another embodiment of the invention. In this decision tree an initial decision is again based on the area stenosis and if the stenosis is greater than 50% a recommendation to stent the area is output. If the stenosis is less than 50% the flow resistance is used in the decision. If the flow resistance is greater than 30% of the vessel without a lesion a recommendation to stent is output. If the flow resistance is less than 30% of the vessel without a lesion a recommendation not to stent is output.

As mentioned above with regard to Figure 5, the thresholds used in figures 6 and 7 are illustrative and other thresholds may be used.

In case that the flow resistance is ascertained based on the cumulative contributions of the multiple portions of the lesion or multiple adjacent lesions with distinct cross sectional area with respect to the neighbouring portion, for any of the embodiments of the decision trees, the cross sectional area reduction of the vessel may take into account the largest reduction of the vessel cross sectional area in percentage from any of the respective multiple portions of the lesion or multiple adjacent lesions.

Providing a recommendation about whether a stent should be used based on the cross sectional area comprises recommending a stent from a plurality of stents. A stent may be selected which is 10% longer than the length of the lesion. Similarly stents of different cross sectional area can be selected based on the cross sectional area of the vessel itself.

Rather than receiving the intravascular images from a remote imaging source the system may comprise an imaging system. In some examples the system comprises an intravascular ultrasound system and/or an optical coherence tomography system. The imaging system may comprise a motorized pullback device to determine the position at which images are taken. An alternative system for determining the position at which images are taken is to use an X-ray imaging system to determine the position of the catheter tip, for example by tracking a radiopaque marker on the intravascular imaging device. Accordingly, the system of the invention may comprise an X-ray imaging system for determining the position or location of a catheter tip within the anatomy, e.g. the vessel.

The invention may be embodied in a computer implemented method for recommending the use or non-use of a stent, using images from an intravascular imaging system and positions of each image along a longitudinal length of the vessel. The intravascular images of the vessel are received and, from the images, a cross sectional area of the vessel at a plurality of positions along the vessel is determined. From the images and the respective position of the images along the vessel a flow resistance of the vessel is determined and a treatment recommendation based on the cross sectional area of the vessel and the flow resistance of the vessel is output.

The invention may also be embodied in a computer program comprising computer program code means which is adapted, when said computer program is run on a computer, to implement the method as described above.

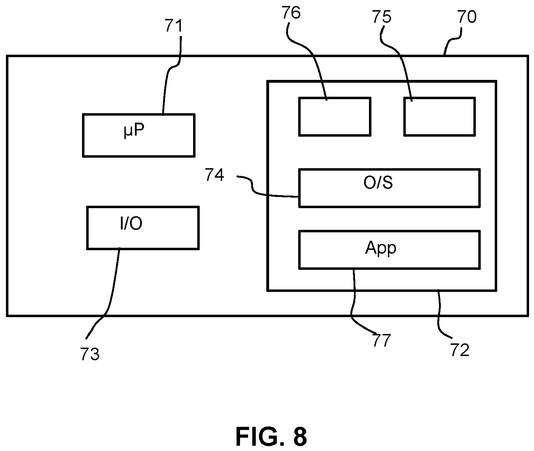

Figure 8 illustrates an example of a computer 70 within which one or more parts of an embodiment may be employed. Various operations discussed above may utilize the capabilities of the computer 70. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 70 includes, but is not limited to, PCs, workstations, laptops, PDAs, palm devices, servers, storages, and the like. Generally, in terms of hardware architecture, the computer 70 may include one or more processors 71, memory 72, and one or more I/O devices 73 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 71 is a hardware device for executing software that can be stored in the memory 72. The processor 71 can be virtually any custom made or commercially available processor, a central processing unit (CPU), a digital signal processor (DSP), or an auxiliary processor among several processors associated with the computer 70, and the processor 71 may be a semiconductor based microprocessor (in the form of a microchip) or a microprocessor.

The memory 72 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 72 may incorporate electronic, magnetic, optical, and/or other types of storage media. Note that the memory 72 can have a distributed architecture, where various components are situated remote from one another, but can be accessed by the processor 71.

The software in the memory 72 may include one or more separate programs, each of which comprises an ordered listing of executable instructions for implementing logical functions. The software in the memory 72 includes a suitable operating system (O/S) 74, compiler 76, source code 75, and one or more applications 77 in accordance with exemplary embodiments. As illustrated, the application 77 comprises numerous functional components for implementing the features and operations of the exemplary embodiments. The application 77 of the computer 70 may represent various applications, computational units, logic, functional units, processes, operations, virtual entities, and/or modules in accordance with exemplary embodiments, but the application 77 is not meant to be a limitation.

The operating system 74 controls the execution of other computer programs, and provides scheduling, input-output control, file and data management, memory management, and communication control and related services. It is contemplated by the inventors that the application 77 for implementing exemplary embodiments may be applicable on all commercially available operating systems.

Application 77 may be a source program, executable program (object code), script, or any other entity comprising a set of instructions to be performed. When a source program, then the program is usually translated via a compiler (such as the compiler 76), assembler, interpreter, or the like, which may or may not be included within the memory 72, so as to operate properly in connection with the O/S 74. Furthermore, the application 77 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 73 may include input devices such as, for example but not limited to, a mouse, keyboard, scanner, microphone, camera, etc. Furthermore, the I/O devices 73 may also include output devices, for example but not limited to a printer, display, etc. Finally, the I/O devices 73 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 73 also include components for communicating over various networks, such as the Internet or intranet.

If the computer 70 is a PC, workstation, intelligent device or the like, the software in the memory 72 may further include a basic input output system (BIOS) (omitted for simplicity). The BIOS is a set of essential software routines that initialize and test hardware at startup, start the O/S 74, and support the transfer of data among the hardware devices. The BIOS is stored in some type of read-only-memory, such as ROM, PROM, EPROM, EEPROM or the like, so that the BIOS can be executed when the computer 70 is activated.

When the computer 70 is in operation, the processor 71 is configured to execute software stored within the memory 72, to communicate data to and from the memory 72, and to generally control operations of the computer 70 pursuant to the software. The application 77 and the O/S 74 are read, in whole or in part, by the processor 71, perhaps buffered within the processor 71, and then executed.

When the application 77 is implemented in software it should be noted that the application 77 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 77 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed image capture and/or processing methods, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 3 and 8 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A system for recommending a treatment of a vessel, the system for use in conjunction with an intravascular imaging system, the intravascular imaging system configured for obtaining a plurality of images of the vessel and providing information on location of each image of the plurality of images along a longitudinal length of the vessel, the system comprising a processor, wherein the processor is adapted to:

   receive a plurality of intravascular images of the vessel at respective locations along the vessel;
   determine, from the plurality of images, at least one cross sectional area of a portion of the vessel;
   determine, from the plurality of images and the respective locations of the plurality of images along the vessel, a flow resistance; and
   output a treatment recommendation based on the at least one cross sectional area of the vessel and the flow resistance.

2. The system according to claim 1, wherein the treatment recommendation is based on at least one of:

   the at least one cross-sectional area being within a first predetermined range; and
   the flow resistance being within a second predetermined range.

3. The system according to any one of the preceding claims, wherein outputting the treatment recommendation is based on a decision tree comprising an assessment of whether the at least one cross-sectional area is within a first predetermined range and the flow resistance is within a second predetermined range.

4. The system according to any of the preceding claims, wherein the processor is configured to:

   determine, from the plurality of images, multiple cross sectional areas of a portion of the vessel at respective locations of the plurality of images along the vessel;
   determine, from the multiple cross sectional areas and the respective locations of the images along the vessel, a cumulative flow resistance; and
   output a treatment recommendation based on the cumulative flow resistance and the at least one of the multiple cross sectional areas.

5. The system according to any one of the preceding claims, wherein the treatment recommendation is based on a reduction of the at least one cross sectional area with respect to the cross sectional area without lesion above a predetermined threshold and based on the flow resistance.

6. The system according to any one of the preceding claims, wherein the flow resistance is calculated as a fraction of the flow resistance of the vessel without lesion.

7. The system according to any one of the preceding claims, wherein the flow resistance R is given by:

$$R = \frac{f}{x^2} R_0$$

where $R_0$ is the flow resistance of the vessel without reduced cross sectional area due to lesion, f is the length of a section with reduced cross sectional area, due to lesion, as a fraction of the length of the vessel and x is the reduced cross sectional area of the vessel as a fraction of the cross sectional area of the vessel being free from lesion.

8. The system according to any one of the preceding claims, wherein outputting the treatment recommendation comprises recommending a stent from a plurality of stents.

9. An intravascular imaging system comprising the system according to any one of the preceding claims, wherein the intravascular imaging system comprises one or more of an intravascular ultrasound system and optical coherence tomography system.

10. The intravascular imaging system according to claim 9, further comprising a motorized pullback device.

11. The intravascular imaging system according to claim 9 or 10, further comprising an X-ray imaging system for determining a location of an intravascular imaging device of the intravascular imaging system.

12. A computer implemented method for recommending a treatment of a vessel, using a plurality of images from an intravascular imaging system and locations of each image of the plurality of images along a longitudinal length of the vessel, the method comprising:

receiving a plurality of intravascular images of the vessel at respective locations along the vessel;
determining, from the plurality of images, at least one cross sectional area of a portion of the vessel;
determining, from the plurality of images and the respective locations of the plurality of images along the vessel, a flow resistance; and
outputting a treatment recommendation based on the cross sectional area of the vessel and the flow resistance.

13. The computer implemented method according to claim 12, wherein outputting a recommendation is based on a decision tree comprising an assessment of whether the at least one of the cross sectional area is within a first predetermined range and the flow resistance is within a second predetermined range.

14. The computer implemented method according to claim 12 or 13, wherein the flow resistance is calculated as a fraction of the flow resistance of the vessel without lesion.

15. A computer program comprising computer program code means which is adapted, when said computer program is run on a computer, to implement the method of any one of claims 12 to 14.

$A_0$

$A_1$

$L_1$

$L_0$

**FIG. 1**

$A_0$

$A_2$

$L_2$

$L_0$

**FIG. 2**

30 → 10

**FIG. 3**

100

Receive plurality of intravascular images — 110

Determine cross sectional area in a plurality of images — 120

Determine flow resistance — 130

Output treatment recommendation — 140

**FIG. 4**

FIG. 5

Area Stenosis

A>50%                 A<50%

Flow resistance                    Flow resistance

R>20%       R<20%          R>30%       R<30%

Stent          No stent         Stent         No stent

## FIG. 6

**FIG. 7**

**FIG. 8**

Europäisches
Patentamt

European
Patent Office

Office européen
des brevets

**EUROPEAN SEARCH REPORT**

Application Number

EP 22 15 5099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/344174 A9 (SCHMITT JOSEPH M [US]; FRIEDMAN JOEL M [IS] ET AL.) 6 December 2018 (2018-12-06) * paragraphs [0003] – [0013] * * paragraphs [0057] – [0059] * * paragraphs [0076] – [0091] * * paragraphs [0110] – [0116] * * figures * | 1-15 | INV. A61B5/026 ADD. A61B5/00 |
| X | US 2015/272448 A1 (FONTE TIMOTHY A [US] ET AL) 1 October 2015 (2015-10-01) * paragraphs [0125] – [0126] * * paragraphs [0152] – [0153] * * paragraphs [0291] – [0294] * * paragraphs [0325] – [0328] * | 1,2,5,7, 8,12,13, 15 | |
| A | WO 2017/199245 A1 (CATHWORKS LTD [IL]) 23 November 2017 (2017-11-23) * page 1, lines 6-15 * * page 27, line 25 – page 28, line 4 * * figures * | 4,6,14 | |
| A | US 2014/142398 A1 (PATIL NITIN [US] ET AL) 22 May 2014 (2014-05-22) * paragraph [0331] * * paragraphs [0481] – [0493] * | 10 | TECHNICAL FIELDS SEARCHED (IPC) A61B G16H |
| A | US 2019/110776 A1 (YU BO [CN] ET AL) 18 April 2019 (2019-04-18) * paragraphs [0026] – [0029] * | 11 | |
| A | WO 2021/016071 A1 (CATHWORKS LTD [US]) 28 January 2021 (2021-01-28) * page 16, line 27 – page 19, line 2 * | 4 | |

The present search report has been drawn up for all claims

| Place of search | Date of completion of the search | Examiner |
|---|---|---|
| The Hague | 5 July 2022 | Bataille, Frédéric |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 22 15 5099

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2022

| Patent document cited in search report | | Publication date | Patent family member(s) | | Publication date |
|---|---|---|---|---|---|
| US 2018344174 | A9 | 06-12-2018 | US 2014276011 A1 | | 18-09-2014 |
| | | | US 2019380594 A1 | | 19-12-2019 |
| | | | US 2022039667 A1 | | 10-02-2022 |
| | | | US 2022039668 A1 | | 10-02-2022 |
| | | | US 2022087544 A1 | | 24-03-2022 |
| | | | US 2022095932 A1 | | 31-03-2022 |
| | | | US 2022095933 A1 | | 31-03-2022 |
| | | | US 2022095934 A1 | | 31-03-2022 |
| US 2015272448 | A1 | 01-10-2015 | EP 3127026 A1 | | 08-02-2017 |
| | | | JP 6378779 B2 | | 22-08-2018 |
| | | | JP 6634123 B2 | | 22-01-2020 |
| | | | JP 6905574 B2 | | 21-07-2021 |
| | | | JP 2017512577 A | | 25-05-2017 |
| | | | JP 2018196742 A | | 13-12-2018 |
| | | | JP 2020044375 A | | 26-03-2020 |
| | | | US 9087147 B1 | | 21-07-2015 |
| | | | US 2015272448 A1 | | 01-10-2015 |
| | | | US 2015324545 A1 | | 12-11-2015 |
| | | | US 2016180055 A1 | | 23-06-2016 |
| | | | US 2017220760 A1 | | 03-08-2017 |
| | | | US 2017329930 A1 | | 16-11-2017 |
| | | | WO 2015153362 A1 | | 08-10-2015 |
| WO 2017199245 | A1 | 23-11-2017 | EP 3457930 A1 | | 27-03-2019 |
| | | | JP 7036742 B2 | | 15-03-2022 |
| | | | JP 2019523029 A | | 22-08-2019 |
| | | | JP 2022071117 A | | 13-05-2022 |
| | | | US 2018184911 A1 | | 05-07-2018 |
| | | | US 2019365246 A1 | | 05-12-2019 |
| | | | US 2021361174 A1 | | 25-11-2021 |
| | | | WO 2017199245 A1 | | 23-11-2017 |
| US 2014142398 | A1 | 22-05-2014 | NONE | | |
| US 2019110776 | A1 | 18-04-2019 | CN 107730540 A | | 23-02-2018 |
| | | | US 2019110776 A1 | | 18-04-2019 |
| WO 2021016071 | A1 | 28-01-2021 | EP 3998939 A1 | | 25-05-2022 |
| | | | WO 2021016071 A1 | | 28-01-2021 |

EPO FORM P0459

For more details about this annex : see Official Journal of the European Patent Office, No. 12/82